# EUROPEAN PATENT SPECIFICATION

(11) **EP 4 565 255 B1**
(45) Date of publication and mention of the grant of the patent: **07.10.2026**
(21) Application number: 23757837.2
(22) Date of filing: 04.08.2023
(51) Int. Cl.: A61K 38/18, A61P 27/16, A61P 25/00

(54) **INTRANASAL ADMINISTRATION OF NGF FOR THE TREATMENT OF SENSORINEURAL HEARING LOSS**
INTRANASALE VERABREICHUNG VON NGF ZUR BEHANDLUNG VON SENSORINEURALEM HÖRVERLUST
ADMINISTRATION INTRANASALE DE NGF POUR LE TRAITEMENT DE LA PERTE AUDITIVE NEUROSENSORIELLE

(30) Priority: 05.08.2022 EP 22188990
(43) Date of publication of application: 11.06.2025
(73) Proprietor: Dompé farmaceutici SpA, 20122 Milano (IT)
(72) Inventor: CASTELLI, Vanessa, 67100 L'Aquila (IT); ARAMINI, Andrea, 67100 L'Aquila (IT); BRANDOLINI, Laura, 67100 L'Aquila (IT); COCCHIARO, Pasquale, 80100 Napoli (IT); ROMEO, Tiziana, 67100 L'Aquila (IT); DETTA, Nicola, 80131 Napoli (IT); APPARENTE, Lucia, 80131 Napoli (IT); MATTIOLI, Simone, 80131 Napoli (IT); CATTANI, Franca, 67100 L'Aquila (IT); MANTELLI, Flavio, 67100 L'Aquila (IT); ALLEGRETTI, Marcello, 67100 L'Aquila (IT)
(74) Representative: Dompé farmaceutici Spa
(86) International application number: PCT/EP2023/071698
(87) International publication number: WO 2024/028487

(56) References cited:
- WO-A1-2013/011536
- WO-A1-2018/215414
- WO-A1-2019/207106
- WO-A1-2022/105847
- WO-A2-2018/140792
- US-A1- 2019 022 101
- SIMONA CAPSONI ET AL: "Intranasal “painless” Human Nerve Growth Factors Slows Amyloid Neurodegeneration and Prevents Memory Deficits in App X PS1 Mice", PLOS ONE, vol. 7, no. 5, 30 May 2012 (2012-05-30), pages e37555, XP055310608, DOI: 10.1371/journal.pone.0037555
- SALVINELLI F. ET AL: "Endogenous nerve growth factor stimulation: effects on auditory pathway neural cells in a mouse model.", 1 January 2018 (2018-01-01), XP055972429, Retrieved from the Internet <URL:https://www.europeanreview.org/wp/wp-content/uploads/7013-7019.pdf> [retrieved on 20221018]
- MANNI LUIGI ET AL: "Intranasal Delivery of Nerve Growth Factor in Neurodegenerative Diseases and Neurotrauma", FRONTIERS IN PHARMACOLOGY, vol. 12, 16 November 2021 (2021-11-16), XP055972509, DOI: 10.3389/fphar.2021.754502

## Description

### FIELD OF THE INVENTION

The present invention relates to NGF or a mutein thereof having the aminoacid sequence as specified in the appendend set of claims for use in the prevention and treatment of sensorineural hearing loss in a subject, wherein said NGF or mutein is administered intranasally to the subject.

### STATE OF THE ART

Hearing loss is the fourth leading cause of disability worldwide, affecting 430 million people, over 5% of the of the world's population. It is estimated that by 2050 over 700 million people will have disabling hearing loss (World Health Organization, 2021 https://www.who.int/news-room/fact-sheets/detail/deafness-and-hearing-loss.).

The term hearing loss refers to a vast array of hearing disorders that can be classified into two main classes: (i) conductive hearing loss, wherein the hearing loss results from obstruction or disease of the outer or middle ear that prevents transmission of sound energy to the inner ear, and (ii) sensorineural hearing loss (SNHL), which constitute 90% of all cases of hearing loss, wherein the hearing loss results from damage to the inner ear and the auditory nerve. In particular, sensorineural hearing loss is mainly characterized by the degeneration of two types of cells: cochlear hair cells, which are the primary mechanoreceptors converting sound energy into neural signals, and/or auditory nerve neurons, the spiral ganglion cells (SGCs), that transmit signals from cochlear hair cells to the auditory nuclei of the brain stem through the auditory nerve.

Sensorineural hearing loss is usually initiated by degeneration of hair cells.

The above two types of cells are strictly connected, and a degeneration of sensory hair cells results in a decrease in synaptic signals stimulating spiral ganglion neurons, leading to neural degeneration (cochlear synaptopathy) (Cunningham al., N. Engl. J. Med. 2017, 377(25): 2465-2473).

The degeneration of hair cells in sensorineural hearing loss may have genetic or non-genetic etiology. Non-genetic factors include noise exposure, viral or bacterial infections, ototoxic chemicals such as the chemotherapeutic drug cisplatin or aminoglycoside antibiotics, autoimmune diseases and aging (Liu et al, Front. Neurosci. 2022, 16: art. 867453).

Despite the significant impact of hearing loss on all segments of the population, the only approved treatment option at the moment is cochlear cochlear implant (CI) auditory prosthesis, which functions by directly stimulating spiral ganglion cells (SGCs) soma and probably their central axons, delivering partial restoration of sensory organ function in patients. On the contrary, no pharmacological therapies that have been approved by FDA for the prevention and treatment of this family of disorders. In clinical practice, non-genetic SNHL is usually treated by administration of corticosteroids, in particular dexamethasone and prednisolone. However, this treatment has not proven to lead to satisfactory outcomes.

It is therefore strongly felt the need to develop new, more effective, pharmacological therapies aimed et the inner ear and able to prevent damage or replenish hairs cells and neuron population in the cochlea and safeguarding and resotoring hearing function.

Nerve growth factor (NGF) is a member of the neurotrophin family that is essential for viability, differentiation, and maintenance of nerve cells.

NGF has been identified in a number of preclinical and clinical studies as a promising therapeutic approach for the treatment of SNHL (Gao et al, Clinical and Experimental Otorhinolaryngology 2017, 10(4): 303-308).

As an alternative to NGF, also muteins of NGF with one or more mutations amino acid sequence have been developed that maintain the neurotrophic and neuroprotective properties of wild-type NGF but show a reduced algogenic activity, thereby avoiding the side effect of increased nociception encountered with wild type NGF.

However, the major challenge for the effective use of NGF or muteins thereof in the prevention or treatment of hearing disorders is the delivery of the protein to the inner ear compartment.

Tha delivery of drugs, particularly proteins as NGF and its muteins to the inner ear compartment is hindered by the presence of several protective barriers.

In details, the blood labyrinth barrier (BLB), which separates the inner ear fluids from blood circulation, limits the possibility of systemic delivery. At the same time, delivery by local administration into the ear is hindered by the barriers between different compartments of the ear, the tympanic membrane (TM), between the outer and middle ear and the round window membrane (RWM), between the middle and inner ear.

To achieve greater drug concentrations in the middle ear, intratympanic injection has been used to deliver active molecules directly into the middle ear for subsequent diffusion into the inner ear across the RWM. However, this delivery route requires an invasive surgical procedure, which may distress patients and carries the risk of permanent tympanic membrane perforation.

WO 2018/215414 A1 relates to the use of neurotrophins, including NGF, in the treatment of hearing disorders related to hearing loss using the transtympanic route of administration.

WO 2018/140792 A2 relates to compositions and methods for the treatment of otic diseases or conditions with non-natural neurotrophic agent compositions and formulations administered to an individual afflicted with an otic disease or condition, through direct application of these compositions and formulations onto or via perfusion into the targeted auris structure(s).

### SUMMARY OF THE INVENTION

The present inventors have now surprisingly found that NGF or its biologically active muteins can be delivered at effective concentrations into the inner ear by intranasal administration.

Accordingly, a first object of described herein is NGF or a mutein thereof for use in the prevention or treatment of sensorineural hearing loss in a subject, wherein said NGF or mutein is administered intranasally to said subject.

A further object described herein is a pharmaceutical composition comprising NGF or a mutein thereof, for use in the prevention or treatment of sensorineural hearing loss in a subject, wherein said composition is administered intranasally to said subject. The invention is defined in the claims.

### BRIEF DESCRIPTION OF THE FIGURES

Figure 1 shows the concentration of NGF in cochlear perilymph at 2 hours post-treatment in rhNGF- treated (NGF treated animals) and PBS-treated (No treated animals) animals, measured as described in Example 1.
Figure 2 shows the concentration of NGF in cochlear perilymph at 12 hours in PBS-treated (PBS) animals and at 12 hours, 24 hours, and 48 hours post-treatment in rhNGF-treated (rhNGF) animals, measured as described in Example 1.
Figure 3 shows the baseline ABRs threshold frequencies, measured before any treatment at different in the three experimental groups of mice: group 1 (SAMR1), group 2 (SAMP8+ vehicle) or group 3 (SAMP8 +rhNGF), as described in Example 2.
Figure 4 shows the baseline DPOAE amplitudes at different frequencies, measured before any treatment in the three experimental groups of mice: group 1 (SAMR1), group 2, (SAMP8+ vehicle) or group 3 (SAMP8 +rhNGF), as described in Example 2.
Figure 5 shows the ABRs threshold at different frequencies, measured after 2 months of treatment in the three experimental groups of mice: group 1 (SAMR1), group 2 (SAMP8+ vehicle) or group 3 (SAMP8 +rhNGF), as described in Example 2.
Figure 6 shows the DPOAE amplitudes at different frequencies, measured after 2 months of treatment in the three experimental groups of mice: group 1 (SAMR1), group 2 (SAMP8+ vehicle) or group 3 (SAMP8 +rhNGF), as described in Example 2.
Figure 7 shows the plasma prestin concentration, measured after 2 months of treatment in the three experimental groups of mice: group 1 (SAMR1), group 2 (SAMP8+ vehicle) or group 3 (SAMP8 +rhNGF), as described in Example 2.
Figure 8 shows the total number of inner hair cells (IHC), measured by the immunohistology analysis of the mid turn of each cochlea in the three experimental groups of mice: group 1 (SAMR1), group 2 (SAMP8+ vehicle) or group 3 (SAMP8 +rhNGF), as described in Example 2.
Figure 9 shows the total number of outer hair cells (OHC), measured by immunohistology analysis of the mid turn of each cochlea in the three experimental groups of mice: group 1 (SAMR1), group 2 (SAMP8+ vehicle) or group 3 (SAMP8 +rhNGF), as described in Example 2.

### DETAILED DESCRIPTION OF THE INVENTION

A first object of the invention is nerve growth factor (NGF) or a mutein thereof for use in the prevention or treatment of sensorineural hearing loss in a subject, wherein said NGF or mutein is administered intranasally to said subject.

The terms "treatment" and "prevention" as used herein refer to the eradication/amelioration or prevention/delay in onset, respectively, of a disorder or of one or more of the symptoms associated thereof.

Preferably, said subject is a human subject.

According to one embodiment, said subject has been diagnosed with a sensorineural hearing loss and said NGF or mutein thereof is for use in the treatment of said sensorineural hearing loss by intranasal administration to said subject.

According to an alternative embodiment, said subject has been identified as being at risk of developing a sensorineural hearing loss and said NGF or mutein thereof is for use in the prevention of said sensorineural hearing loss by intranasal administration to said subject.

Preferably, said sensorineural hearing loss is a sensorineural hearing loss of non-genetic etiology.

Preferably, said sensorineural hearing loss of non genetic etiology is caused by noise exposure, a bacterial or viral infection, the treatment with an ototoxic drug, an autoimmune disease or aging.

Preferably, said ototoxic drug is selected from chemotherapeutic drugs and aminoglycoside antibiotics, more preferably it selected from cisplatin, caroboplatin and gentamicin.

Preferably said NGF is human NGF.

Preferably, said human NGF has the aminoacid sequence of SEQ. ID NO.1 below

SEQ. ID NO.1:
SSSHPIFHRGEFSVCDSVSVWVGDKTTATDIKGKEVMVLGEVNINNSVFKQYFFETKC
RDPNPVDSGCRGIDSKHWNSYCTTTHTFVKALTMDGKQAAWRFIRIDTACVCVLSRK
AVR

Alternatively, said human NGF has the aminoacid sequence of SEQ ID NO. 2 below:

SEQ ID NO. 2:
SSSHPIFHRGEFSVCDSVSVWVGDKTTATDIKGKEVMVLGEVNINNSVFKQYFFETKC
RDPNPVDSGCRGIDSKHWNSYCTTTHTFVKALTMDGKQAAWRFIRIDTACVCVLSRK
AVRRA

Alternatively, said human NGF is a mixture of NGFs having sequences of SEQ ID NO. 1 and SEQ ID NO. 2.

The human NGF of SEQ ID NO. 2 have an aminoacid sequence that only differ from the NGF of SEQ ID NO. 1 for the presence of two additional amminoacids at the C-terminus. Both forms of NGF are found in human cells and therefore are considered as wild type human NGF.

Therefore, when referring to "human NGF" or "wild type human NGF" in the present application, it is meant a human NGF of SEQ ID NO. 1 or SEQ ID NO. 2.

Preferably, said NGF is produced by recombinant DNA technology, preferably it is a human recombinant NGF (rhNGF). Methods of producing rhNGF are known to the person skilled in the art, for example those described in WO0022119A1 and WO2013092776A1.

Preferably, said NGF has a purity higher than 70%, more preferably higher than 80%, higher than 90%, higher than 95%, higher than 98% or higher than 99%. The purity of NGF may be determined by conventional means known to those skilled in the art, for example by HPLC analysis.

The term "mutein of NGF" refers to a biologically active mutein of NGF, meaning a NGF protein having an aminoacid sequence with one or more aminoacid mutations, preferably substitutions, such that the therapeutic activity of wild type NGF is maintained.

Preferably, said mutein is a mutein of wild type human NGF.

Particularly preferred for use according to the invention, is a mutein of NGF, wherein the mutein is characterized by more than 70 %, more preferably more than 80 %, even more preferably more than 90 %, and most preferably more than 95 % sequence identity with wild type human NGF.

Preferably, the mutein is a mutein of wild type human NGF characterized by at least one mutation, preferably aminoacid substitution of proline at position 61 by another amino acid, in the sequence of wild type human NGF. In a particularly preferred embodiment, proline at position 61 is substituted by serine.

Preferably, the mutein is a mutein of human NGF, characterized by at least one mutation of the amino acid sequence associated with reduced nociceptive activity. More preferably said mutein is characterized by at least one mutation, preferably amino acid substitution, at any of positions 95-101 of wild type human NGF. Even more preferably, said mutein is characterized by substitution of the arginine in position 100 of wild type human NGF. Most preferably, arginine at position 100 of wild type human NGF is substituted by glutamic acid.

Particularly preferred muteins according to the invention have the aminoacid sequences of SEQ ID NO. 3-6 below
SEQ ID NO. 3: 
SSSHPIFHRGEFSVCDSVSVWVGDKTTATDIKGKEVMVLGEVNINNSVFKQYFFETKC
RDPNPVDSGCRGIDSKHWNSYCTTTHTFVKALTMDGKQAAWEFIRIDTACVCVLSRKA
VR
SEQ ID NO. 4: 
SSSHPIFHRGEFSVCDSVSVWVGDKTTATDIKGKEVMVLGEVNINNSVFKQYFFETKC
RDPNPVDSGCRGIDSKHWNSYCTTTHTFVKALTMDGKQAAWEFIRIDTACVCVLSRKA
VRRA
SEQ ID NO. 5: 
SSSHPIFHRGEFSVCDSVSVWVGDKTTATDIKGKEVMVLGEVNINNSVFKQYFFETKC
RDSNPVDSGCRGIDSKHWNSYCTTTHTFVKALTMDGKQAAWEFIRIDTACVCVLSRKA
VR
SEQ ID NO. 6:

SSSHPIFHRGEFSVCDSVSVWVGDKTTATDIKGKEVMVLGEVNINNSVFKQYFFETKC
RDSNPVDSGCRGIDSKHWNSYCTTTHTFVKALTMDGKQAAWEFIRIDTACVCVLSRKA
VRRA

The above described muteins are particularly advantageous for use according to the invention since they maintain the same bioactivity than wild type human NGF but are able to induce a lower nociceptive sensitivity compared to the corresponding wild type human NGF. Preferably, said mutein of human NGF is produced by recombinant DNA technology. Methods of producing muteins of rhNGF according to the invention by recombinant DNA technology are known to the person skilled in the art, for example those described in WO2019/207106.

Preferably, the NGF or mutein for use according to the invention is administered to the subject from one to three times a day for a period of treatment of between 7 and 300 days, preferably between 60 and 240 days, more preferably between 100 and 200 days. Preferably, the amount of NGF or mutein thereof per each administration is between 5 µg and 1 mg, more preferably between 10 µg and 400 µg, even more preferably between 15 µg and 200 µg.

In case of use for the treatment of SNHL, the effective amount of said NGF or mutein used in each administration, the duration of the treatment and the number of administrations for day are selected by the skilled person on the basis of the characteristics of the subject to be treated, the severity of the SNHL and on the basis of the auditory tests carried out during the treatment.

In case of use for the prevention of SNHL, the effective amount of said NGF or mutein used in each administration, the duration of the treatment and the number of administrations per day are selected by the skilled man on the basis of the characteristics of the subject at risk of developing SNHL and the clinical evaluation of the entity and duration of the risk of developing hearing loss as a result of one of said causes.

The evaluation of the above factors is within the knowledge and expertise of the skilled person.

A further object of the present invention relates to a pharmaceutical composition for intranasal administration comprising the NGF or mutein as described above and at least one pharmaceutically acceptable excipient suitable for intranasal use.

Preferably, the pharmaceutical composition for intranasal administration of the invention is a liquid intranasal composition.

Preferably, the pharmaceutical composition according to the present invention comprises an effective amount of the NGF or mutein as described above and at least one pharmaceutically acceptable excipient suitable for intranasal use, preferably selected from solvents, thickening agents, mucoadhesive agents, buffers, antioxidants, preservatives, and penetration enhancers.

Preferably, the concentration of said NGF or mutein in the liquid intranasal composition according to the invention is between 5 µg/ml and 1 mg/ml, more preferably between 10 µg/ml and 400 µg/ml, even more preferably between 15 µg/ml and 200 µg/ml.

Preferably, said solvent is water.

Preferably, said mucoadhesive agent is glycerol, more preferably at a concentration between 0.05 % w/v and 0.2% w/v, more preferably of 0.1% w/v.

Preferably, said antioxidant is methionine, more preferably at a concentration between 0.005 mg/ml and 0.02 mg/ml, more preferably of 0.01 mg/ml.

Preferably said surfactant is Kolliphor P188 (Poloxamer 188), more preferably at a concentration between 0.05 % w/v and 0.2% w/v, more preferably of 0.1% w/v.

The term "Kolliphor P188", as used herein, refers to Poloxamer 188, a block copolymer that is a synthetic copolymer of ethylene oxide and propylene oxide represented by the following chemical structure: 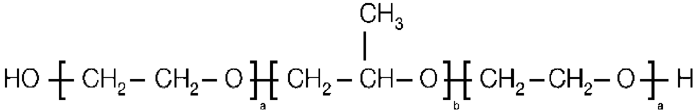 where in a and b blocks have the following values:

| **Kolliphor^{®}** | **Poloxamer** | **a** | **b** |
|---|---|---|---|
| P 188 | 188 | 80 | 27 |

Preferably, said buffer is phosphate buffer.

Preferably, said penetration enhancer is n-Dodecyl-β-D-maltoside, more preferably at a concentration between 0.1 % w/v and 1% w/v, more preferably of 0.5% w/v.

A particularly preferred liquid intranasal composition according to the invention comprises, preferably consists of, said NGF or mutein thereof, sodium chloride, phosphate buffer and water.

Another particularly preferred liquid intranasal composition according to the invention comprises, preferably consists of, said NGF or mutein thereof as described above, sodium chloride, phosphate buffer, Kolliphor P188 (Poloxamer 188), L-Methionine, and water.

Another particularly preferred liquid intranasal composition according to the invention comprises, preferably consists of said NGF or mutein, sodium chloride, phosphate buffer, Kolliphor P188 (Poloxamer 188), L-Methionine, Glycerol, n-Dodecyl-β-D-maltoside and water.

Preferably, the liquid intranasal composition according to the invention comprises, preferably consists of, the following components:
- NGF or a mutein thereof, as described above, preferably at a concentration between 5 µg/ml and 1 mg/ml, more preferably between 10 µg/ml and 400 µg/ml, even more preferably between 15 µg/ml and 200 µg/ml,
- NaH2PO4 * H2O, preferably at a concentration between 5 and 8 mg/ml, more preferably of 6.9 mg/mL,
- NaCl, preferably at a concentration between 5 and 6.5 mg/ml, more preferably of 5.84 mg/mL,
- Poloxamer 188, preferably at a concentration between 0.05 % w/v and 0.2% w/v, more preferably of 0.1% w/v,
- L-Methionine, preferably at a concentration between 0.005 mg/ml and 0.02 mg/ml, more preferably of 0.01 mg/ml,
- Optionally, n-Dodecyl-β-D-maltoside, preferably at a concentration between 0.1 % w/v and 1% w/v, more preferably of 0.5% w/v, and/or glycerol, preferably at a concentration between 0.05 % w/v and 0.2% w/v, more preferably of 0.1% w/v,
- Water.

The pharmaceutical composition according to the invention may be suitably formulated using appropriate methods known in the art or by the method disclosed in Remington's Pharmaceutical Science (recent edition), Mack Publishing Company, Easton Pa.

The pharmaceutical composition of the invention is for use in the prevention or treatment of sensorineural hearing loss in a subject, as above described, wherein said composition is administered intranasally to the subject.

The invention will be further described in the following examples.

### Example 1

The bioavailability of rhNGF (having the sequence of SEQ ID NO.1) in the inner ear (perilymph) after a single intranasal administration was evaluated in C57BL6 male mice 10 weeks old.

The mice were randomly divided in six groups: two control groups (n=3) which received vehicle and whose cochlear perilymphs were sampled two hours and twelve hours after vehicle administration; four treated groups which received a single administration of 10µL rhNGF (SEQ ID NO.1) solution at a concentration of 2 mg/mL in phosphate buffer, and sampling at 2 h (n=4), 12h (n=4), 24h (n=4) and 48h (n=4). For the intranasal treatment, the tip of the filled pipette was placed near the mouse's left nostril, kept at 45 degree angle, and a droplet of the content released so that it was immediately inhaled by the mouse. A second droplet was released to the mouse so that it could be inhaled through the same nostril about 2-3 sec later. After administration, the mice were held in this position for 15 sec.

All mice were sacrificed by cervical dislocation and the tympanic bullas were isolated. Each tympanic bulla was opened, the cochlea was cleaned with PBS and 1 µl of cochlear perilymph was sampled from each bulla using a capillary micropipette and immediately frozen in a low-bidding Eppendorf tube and stored at -20 °C before ELISA analysis.

The cochlear perilymph samples were diluted at 1/10 and analyzed by ELISA (Novus Biological, Ref. NBP2-62776). Duplicates were performed with the right and left cochlea The manufacturer's protocol provided was optimized. Standard references were diluted at 1000, 500, 250, 125, 62.5, 31.25, 15.63 and 0 pg/mL to perform the standard curve. The Standard working solution of different concentrations was added to the first two columns: Each concentration of the solution was added into two wells side by side and the samples to other wells (10 µl/well). The plate was covered with sealer provided in the kit and incubated for 90 min at 37°C. Then, the liquid of each well was removed and immediately 100 µL of Biotinylated Detection Ab working solution was added to each well. The plate was again covered with sealer provided in the kit and incubated for 1 hour at 37°C. After incubation, the solution from each well was aspirated and 350 µL of wash buffer was added and aspirated to each well. This wash step was repeated 3 times. 100 µL of HRP Conjugate working solution was added to each well. The plate was again covered with sealer provided in the kit and incubated for 30 min at 37°C. After washing, 90 µL of Substrate Reagent was added to each well and incubated for 15 min at 37°C. Finally, 50 µL of Stop Solution was added to each well and the optical density of each well was immediately determined using a micro-plate reader set to 450 nm.

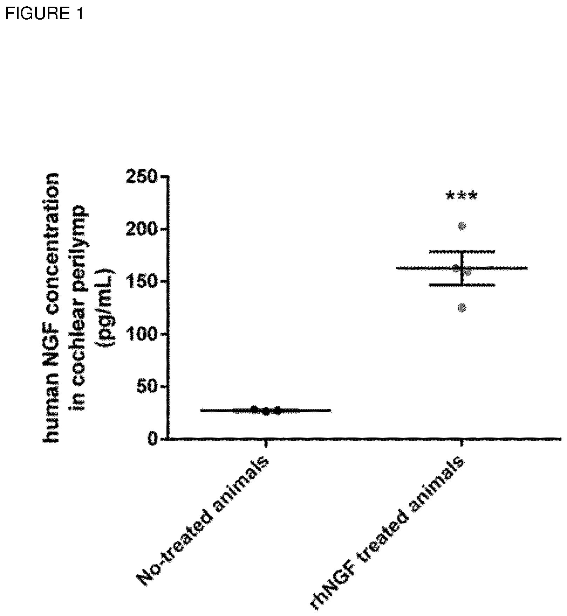

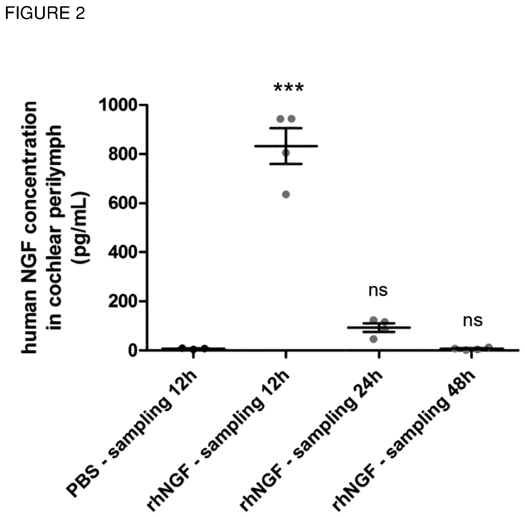

The results of the analysis are reported in Figure 1 and 2.

Descriptive statistics by groups are expressed as mean ± SEM for continuous variables. Statistical significances were determined using t-test to compare treated versus control non-treated animals. Statistical analyses were performed using GraphPad Prism version 5.02 for Windows (GraphPad Software, La Jolla California USA). A P value of less than 0.05 was considered significant.

As can be seen from Figures 1 and 2, a significant increase of human NGF concentration was observed in perilymph of compound treated groups compared to the control non-treated groups. Two hours after single NGF administration by intranasal route, the compound was detected in perilymph at a mean of 162.7 pg/mL while in the control non-treated groups a mean concentration of 27.3 pg/ml was detected (Fig.1); the peak concentration was reached twelve hours post-treatment when the mean was 832.201 pg/ml in treated groups vs 7.331 in the control no-treated groups (Fig.2).

The data obtained demonstrate that intranasal administration of NGF is an effective way of delivering the protein to the inner ear.

### Example 2

The efficacy of rhNGF (having the sequence of SEQ ID NO.1) administered by intranasal route was tested in a preclinical age-related hearing loss (ARHL) mouse model, the Senescence Accelerated Mouse Prone (SAMP8) strain. This strain has been widely used in aging research to study phenotypes such as peripheral neuropathy, hearing loss, immune dysfunction, osteoporosis, blindness and brain atrophy. The premature SAMP8 senescence involves oxidative stress, altered levels of antioxidant enzymes and increase of senescent cells, leading to chronic inflammation closely mimicking human senescence. Senescence-Accelerated Resistant (SAMR1) mice were used in the experiments as normal aging control.

SAMR1 and SAMP8 male mice 1 month old were purchased from Envigo, France (n=24). Mice were housed in macrolon cages with filter hoods, in a room where the air is continuously filtered, thereby avoiding contamination. During experiments, paired animals were caged at a constant temperature with a day/night cycle of 12/12 hours.

Mice were then divided in three groups, each treated as follows:
Group 1: SAMR1 mice (SAMR1) without treatment (negative control n=8),
Group 2: SAMP8 mice treated with vehicle (SAMP8+ vehicle) (n=8),
Group 3: SAMP8 mice treated with 10 µl of a solution rhNGF at 2mg/ml in Saline buffer(vehicle), administered by intranasal route (SAMP8 +rhNGF) (n=8).

Evaluation of auditory brainstem response (ABRs) and distortion product otoacoustic emission (DPOE) was carried out on all animals at day 1 before any treatment, following the procedure described below.

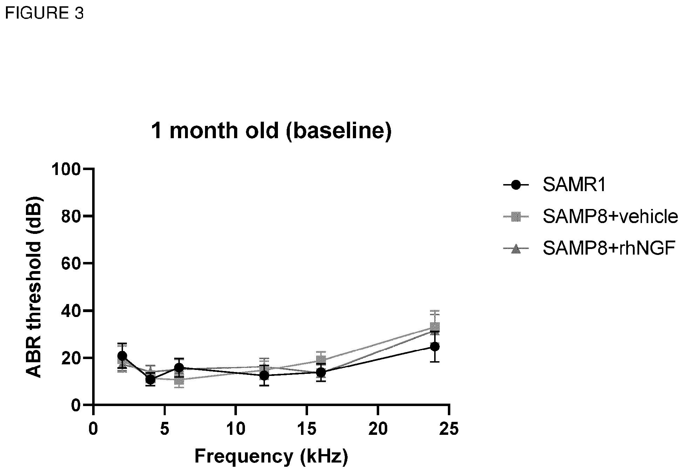

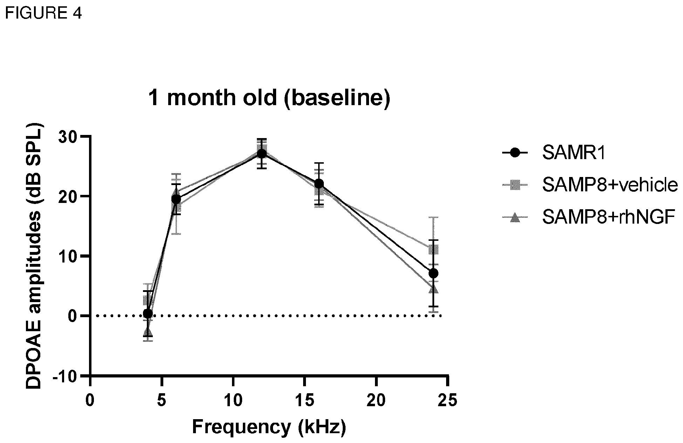

As shown in Figures 3 and 4, similar ABR thresholds and DPOAE amplitudes were observed at the baseline for the three groups at the analyzed frequencies demonstrating the absence of hearing impairment at this time-point.

The intranasal administration was carried out once a day following the same procedure described in Example 1 starting at one month of age of the animals for 60 days.

No mortality nor pathological clinical sign were observed during the study.

At day 60, corresponding to 3 months of age of the animals, these were subjected to a number of tests, described below, in order to determine the effect of the treatment on hearing impairment.

Statistical significances of the data obtained were determined using 2-way or 1-way ANOVA, followed by a Bonferroni or Turkey' multiple comparisons post hoc test, allowing comparisons between groups, assuming the normal distribution of the variable and the variance homoscedasticity. Statistical analyses were performed using GraphPad Prism version 5.02 for Windows (GraphPad Software, La Jolla California USA). A P value of less than 0.05 was considered significant. Descriptive statistics by groups was expressed as mean ± SEM for continuous variables.

### Auditory brainstem responses (ABRs)

ABRs are electric potentials recorded from scalp electrodes, and the first ABR wave represents the summed activity of the auditory nerve fibers contacting the inner hair cells. For ABR studies, mice were anesthetized using ketamine/xylazine mixture, and body temperature was regulated using a heating pad at 37 °C. Then, earphones were placed in the left ear of each mouse, an active electrode was placed in the vertex of the skull, a reference electrode under the skin of the mastoid bone and a ground electrode in the neck skin. The stimuli consisted of tone pips of five frequencies (2 kHz, 4 kHz, 6 kHz, 12 kHz, 16 kHz and 24 kHz) at various sound levels (from 0 to 90 dB) ranging to cover the mouse auditory frequency range. ABR measures of each animal were performed individually and using OtoPhyLab system. Evoked potentials were extracted by the signal averaging technique for each noise level and ABR thresholds for each frequency were determined using OtoPhyLab software.

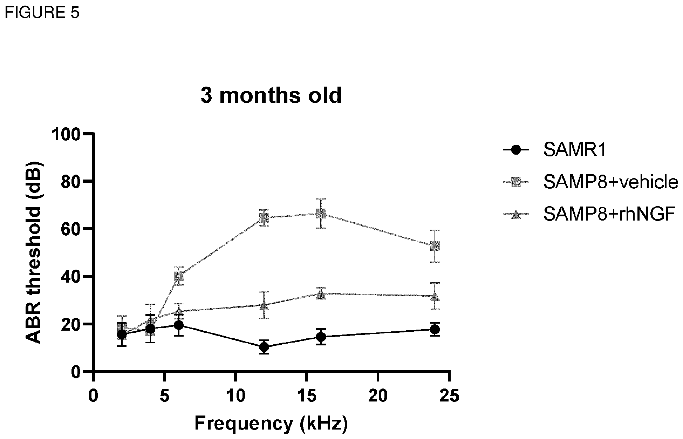

The results obtained for each group of animals are shown in Figure 5.

As expected, significantly higher ABR thresholds were observed for the SAMP8+vehicle group compared to the SAMR1 control group at 6 kHz, 12 kHz, 16 kHz and 24 kHz at 3 months old (Figure 4, SAMP8+vehicle vs SAMR1). The SAMP8 animals treated with rhNGF presented a significantly lower ABR threshold at 12 kHz, 16 kHz and 24 kHz compared to the SAMP8+vehicle group, showing a substantial protective effect of NGF administered intranasally on the hearing impaired induced by aging (Figure 3, SAMP8+NGF vs SAMP8+vehicle).

### Distortion product otoacoustic emission (DPOAE)

DPOAEs are acoustic signals created and amplified by the cochlear epithelium, offering an index of cochlear functions. They are related to outer hair cell (OHCs) health which amplifies sound-evoked cochlear vibrations. They do not depend on IHCs or auditory nerve fibers. For DPOAE measures, mice were anesthetized using ketamine/xylazine mixture and a probe (OtoPhyLab) was inserted into the external left ear canal. The primary tone F2 was set at five frequencies (4 kHz, 6 kHz, 12 kHz, 16 kHz and 24 kHz) at 58 dB. The frequency ratio F2/F1 was set at 1.2. At all frequencies (F2), the input of DPOAE systems was received, digitized, and evaluated using the output of the microphone. The amplitudes of the frequency component at the distortion product frequency were determined and represented.

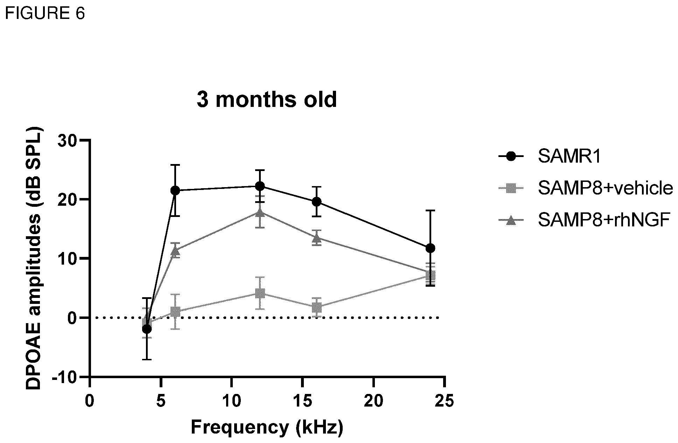

The results obtained are shown in Figure 6.

As expected, significantly lower DPOAE amplitude was observed for the SAMP8+vehicle group compared to the SAMR1 control group at 6 kHz, 12 kHz and 16 kHz at 3 months old (Figure 6, SAMP8+vehicle vs SAMR1). The SAMP8 animals treated with rhNGF presented a significantly higher DPOAE amplitude at 6 kHz, 12 kHz and 16 kHz compared to the SAMP8+vehicle group (Figure 6, SAMP8+rhNGF vs SAMP8+vehicle) confirming a direct or indirect protective role of nerve growth factor administered intranasally on the outer hair cell functionality leading to hearing improvement in ageing animals.

### Prestin quantification

Prestin is a protein recognized as biomarker for cochlear damage. Therefore, the level of this protein was evaluated in the animals at the end of the treatment. For each animal, 2 mL of blood was sampled by cardiac puncture and collected in a tube containing EDTA as anticoagulant. Samples were centrifuged for 15 minutes at 1000 × g (or 3000 rpm) at 2 - 8°C within 30 minutes of collection. The supernatant (plasma) was stored at -80°C until analysis. Prestin quantification for each animal was performed in duplicated by ELISA method (Mybiosource. Ref. SLC26A5. Cat Number. MBS286559).

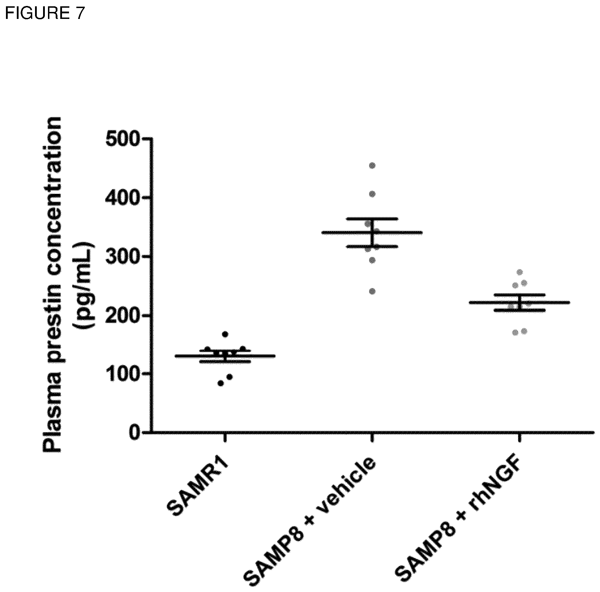

The results obtained are shown in Figure 7.

As expected, a significantly higher plasma prestin concentration was observed in the SAMP8+vehicle group compared to the SAMR1 control group (Figure 7, SAMP8+vehicle vs SAMR1) at 3 months old, indicating cochlear damage and corroborating electrophysiological and otoacoustic readouts. The SAMP8 animals treated with rhNGF presented a significantly lower plasma prestin concentration compared to the SAMP8+vehicle group (Figure 7, SAMP8+rhNGF vs SAMP8+vehicle). Taken together, these data confirm the protective role of nerve growth factor administered intranasally on hearing impairment induced by ageing.

### Cytocochleogram and ribbon synapses

Quantification of the total number of inner hair cells (IHC) and outer hair cells (OHC) was performed by the immunohistology analysis of the mid turn of each cochlea. In details, left cochleae of all animals were dissected out and fixed with paraformaldehyde solution overnight and then, decalcified for 7 days in EDTA. The membranous and sensory spiral containing the organ of Corti were extracted and the hair cells were immunolabeled for anti-Myosin-VIIa. The medial region of the organ of Corti was mounted on glass slides and images were acquired with a confocal microscope and exported in tiff. The total numbers of inner and outer hair cells were counted for the mid segment. For the ribbon synapse immunostaining, the right cochleae of 1 animal/group were sampled, fixed and decalcified as previously stated and the ribbon synapses of the mid segment were immunolabeled for anti-GluR2 and the cochlear neurons using anti-Tuj1. Samples were mounted on glass slides and images were acquired with a confocal microscope and exported in tiff.

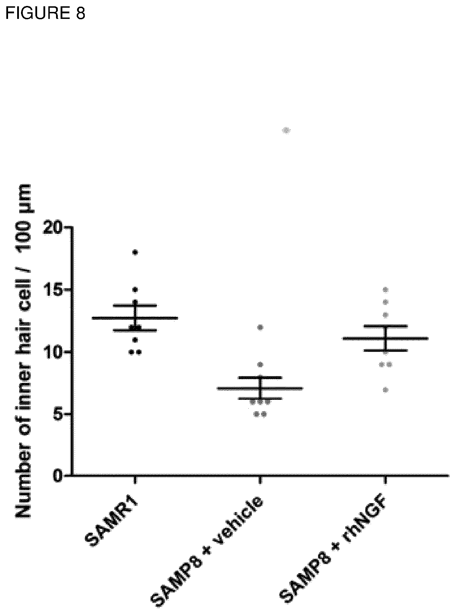

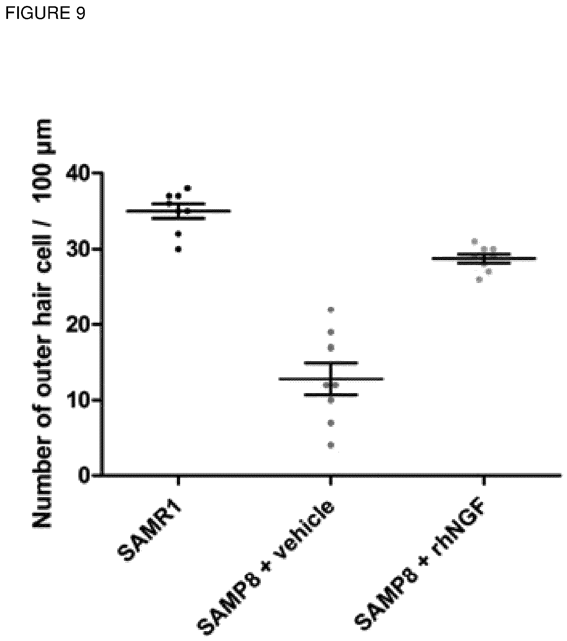

As expected, significantly lower number of IHC and OHC was observed in the SAMP8+vehicle group compared to the SAMR1 control group at 3 months old, confirming the cochlear damage induced by senescence (Figure 8 and 9, respectively, SAMP8+vehicle vs SAMR1). The SAMP8 animals treated with rhNGF presented significantly higher number of IHC and OHC compared to the SAMP8+vehicle group (Figure 7 and 8, respectively, SAMP8+rhNGF vs SAMP8+vehicle). Taken together, these results confirm the positive efficacy of the intranasal administration of nerve growth factor on the age-related hearing loss

### Cochlea scanning electron microscopy imaging

Right cochleae of 3 animals per each group were removed from the temporal bone and fixed in 2.5% glutaraldehyde in PHEM buffer (PIPES 60mM, HEPES free acid 25mM, EGTA 20 mM, MgCl2 2mM) overnight at room temperature. Then, the stria vascularis, tectorial, and Reissner's membranes were removed by microdissection. After rinsing in PHEM buffer, the samples were dehydrated in a graded series of ethanol (30-100%), critical point dried in CO2, coated with gold palladium, observed using a scanning electron microscope Hitachi S4000 and exported in tiff.

Outer and inner hair cell stereocilia morphology was determined by scanning electron microscopy in the mid turn of the cochlea. Outer hair cell loss was confirmed in the SAMP8 animals treated with vehicle whereas no cell loss was observed in SAMR1 control animals or in SAMP8 animals treated with rhNGF. Inner hair cells stereocilia fusion was observed in the SAMP8 animals treated with vehicle whereas normal stereocilia morphology was observed in the SAMP8 animals treated with rhNGF. Morphology of stereocilia of SAMP8+rhNGF group was close that of SAMR1 control animals confirming the protective effect of the intranasal administration of nerve growth factor also from a histological point of view.

### Ribbon synapses imaging

Imaging of ribbon synapses and cochlear neurons was performed by immunohistolabeling of the cochlear mid turn.

As expected, a lower number of ribbons synapses and neurons was observed in cochlea of SAMP8 mouse treated with vehicle compared to SAMR1 mouse at 3 months. A number of ribbon synapses and efferent and afferent cochlear neurons similar to the SAMR1 control animal was observed in the SAMP8 mouse treated with the rhNGF confirming also in this case the protective effect of the intranasal administration of nerve growth factor from a histological point of view.

### Conclusions:

As expected, increase of ABR threshold, decrease of DPOAE amplitude, increase of plasma prestin concentration and significant cochlear hair cell loss was observed in the SAMP8 mice treated with vehicle at 3 months, confirming the presence of hearing impairment due to the accelerated senescence of this mouse model. No differences were observed in the SAMR1 control animals. The SAMP8 mice treated with rhNGF once a day for two months presented significant decrease of ABR threshold, increase of DPOAE amplitude, and decrease of plasma prestin concentration and cochlear hair cell loss at 3 months compared to the vehicle group. Moreover, histological characterization by SEM and immunohistochemistry of cochleae demonstrated the absence of stereocilia fusion of inner hair cells and increase of the number of ribbon synapses and cochlear neurons after compound treatment corroborating electrophysiology, otoacoustic and functional data. Taken together, these results confirm that nerve growth factor administrated by intranasal route presents significant protective efficacy on the age-related hearing impairment in the preclinical model of accelerated senescence SAMP8.

### Example 3

The inventors have prepared the following example pharmaceutical compositions, obtained according to the below-disclosed preparation methods.

### Composition 1

- rhNGF having the sequence of SEQ ID NO.1 (0.6 mg/mL)
- 6.9 mg/mL NaH2PO4 * H2O
- 5.84 mg/mL NaCl
- 1 mg/mL Kolliphor P188
- 5 mg/mL n-Dodecyl-β-D-maltoside
- 0.01 mg/mL L-Methionine
- WFI

In 100 mL of water dissolve 0.69 g NaH2PO4 * H2O, 0.58 g NaCl and 0.1 g of Kolliphor P188. Wait for complete solubilization and the add 0.5 g of n-Dodecyl-β-D-maltoside under magnetic stirring. After the complete solubilization add 0.001 g L-Methionine. The pH was adjusted at 7 with NaOH.

After the complete dissolution the composition was filtered with 0.22µm filter.

The neurotrophin was added to the composition at final concentration 0.6 mg/mL.

### Composition 2

- rhNGF having the sequence of SEQ ID NO.1 (0.6 mg/mL)
- 6.9 mg/mL NaH2PO4 * H2O
- 5.84 mg/mL NaCl
- 5 mg/mL n-Dodecyl-β-D-maltoside
- 0.01 mg/mL L-Methionine
- WFI

In 100 mL of water dissolve 0.69 g NaH2PO4 * H2O and 0.58 g NaCl. Wait for complete solubilization and thenadd 0.5 g of n-Dodecyl-β-D-maltoside under magnetic stirring. After the complete solubilization add 0.001 g L-Methionine. The pH was adjusted at 7 with NaOH.

After the complete dissolution the composition was filtered with 0.22µm filter.

The neurotrophin was added to the composition at final concentration 0.6 mg/mL.

### Composition 3

- rhNGF having the sequence of SEQ ID NO.1 (0.6 mg/mL)
- 6.9 mg/mL NaH2PO4 * H2O
- 5.84 mg/mL NaCl
- 1 mg/mL Kolliphor P188
- 5 mg/mL n-Dodecyl-β-D-maltoside
- 2 mg/mL glycerol
- 0.01 mg/mL L-Methionine
- WFI

In 100 mL of water dissolve 0.69 g NaH2PO4 * H2O, 0.58 g NaCl and 0.1 g of Kolliphor P188. Wait for complete solubilization and the add 0.5 g of n-Dodecyl-β-D-maltoside under magnetic stirring. Then add 0.2 g of glycerol. After the complete solubilization of all excipient add 0.001 g L-Methionine. The pH was adjusted at 7 with NaOH.

After the complete dissolution the composition was filtered with 0.22µm filter.

The neurotrophin was added to the composition at final concentration 0.6 mg/mL.

### Composition 4

- rhNGF having the sequence of SEQ ID NO.1 (0.6 mg/mL)
- 6.9 mg/mL NaH2PO4 * H2O
- 5.84 mg/mL NaCl
- 1 mg/mL Kolliphor P188
- 2 mg/mL glycerol
- 0.01 mg/mL L-Methionine
- WFI

In 100 mL of water dissolve 0.69 g NaH2PO4 * H2O and 0.58 g NaCl. Wait for complete solubilization and then add 0.5 g of n-Dodecyl-β-D-maltoside under magnetic stirring. Then add 0.2 g of glycerol. After the complete solubilization of all excipients add 0.001 g L-Methionine. The pH was adjusted at 7 with NaOH.

After the complete dissolution the composition was filtered with 0.22µm filter.

The neurotrophin was added to the composition at final concentration 0.6 mg/mL.

## Claims

1. Nerve growth factor (NGF) or a mutein thereof having the aminoacid sequence of SEQ ID No. 3, SEQ ID No. 4, SEQ ID No. 5 or SEQ ID No. 6, for use in the prevention or treatment of sensorineural hearing loss in a subject, wherein said NGF or mutein is administered intranasally to said subject.

2. NGF or mutein for use as claimed in claim 1, wherein said sensorineural hearing loss is of non-genetic etiology.

3. NGF or mutein for use as claimed in claim 2, wherein said sensorineural hearing loss of non-genetic etiology is caused by noise exposure, a bacterial or viral infection, the treatment with an ototoxic drug, an autoimmune disease or aging.

4. NGF or mutein for use as claimed in claims 1 to 3, wherein said NGF is human NGF, more preferably it is recombinant human NGF.

5. NGF or mutein for use as claimed in claim 4, wherein said human NGF has the aminoacid sequence of SEQ ID No. 1 or SEQ ID No. 2.

6. NGF or mutein for use as claimed in claims 1 to 5, wherein said NGF or mutein is administered from one to three times a day for a period of treatment of between 7 and 300 days, preferably between 60 and 240 days, more preferably between 100 and 200 days.

7. NGF or mutein for use as claimed in claims 1 to 5, wherein the amount of NGF per each administration is between 5 µg and 1 mg, more preferably between 10 µg and 400 µg, even more preferably between 15 µg and 200 µg.

8. A pharmaceutical composition comprising NGF or mutein thereof having the aminoacid sequence of SEQ ID No. 3, SEQ ID No. 4, SEQ ID No. 5 or SEQ ID No. 6, and at least one pharmaceutically acceptable excipient, for use in the prevention or treatment of sensorineural hearing loss in a subject, wherein said composition is administered intranasally to said subject.

9. A pharmaceutical composition for use as claimed in claim 8, wherein said sensorineural hearing loss of non-genetic etiology is caused by noise exposure, a bacterial or viral infection, the treatment with an ototoxic drug, an autoimmune disease or aging.

10. A pharmaceutical composition for use as claimed in claim 8 or 9, wherein said NGF or mutein thereof is present in the composition at a concentration between 5 µg/ml and 1 mg/ml, more preferably between 10 µg/ml and 400 µg/ml, even more preferably between 15 µg/ml and 200 µg/ml.

11. A pharmaceutical composition for use as claimed in claims 8 to 10, comprising, preferably consisting of NGF or mutein thereof, sodium chloride, phosphate buffer and water.

12. A pharmaceutical composition for use as claimed in caims 8 to 11, comprising, preferably consisting of:
- NGF or mutein thereof having the aminoacid sequence of SEQ ID No. 3, SEQ ID No. 4, SEQ ID No. 5 or SEQ ID No. 6, preferably at a concentration between 5 µg/ml and 1 mg/ml, more preferably between 10 µg/ml and 400 µg/ml, even more preferably between 15 µg/ml and 200 µg/ml,
- NaH2PO4 * H2O, preferably at a concentration between 5 and 8 mg/ml, more preferably of 6.9 mg/mL,
- NaCl, preferably at a concentration between 5 and 6.5 mg/ml, more preferably of 5.84 mg/mL,
- Poloxamer 188, preferably at a concentration between 0.05 % w/v and 0.2% w/v, more preferably of 0.1% w/v,
- L-Methionine, preferably at a concentration between 0.005 mg/ml and 0.02 mg/ml, more preferably of 0.01 mg/ml,
- Optionally, n-Dodecyl-β-D-maltoside, preferably at a concentration between 0.1 % w/v and 1% w/v, more preferably of 0.5% w/v, and/or glycerol, preferably at a concentration between 0.05 % w/v and 0.2% w/v, more preferably of 0.1% w/v,
- Water.

13. A pharmaceutical composition for use as claimed in claims 8 to 12, wherein said NGF is human NGF, more preferably it is recombinant human NGF.

14. A pharmaceutical composition for use as claimed in claim 13, wherein said human NGF has the aminoacid sequence of SEQ ID No. 1 or SEQ ID No. 2.

## Patentansprüche

1. Nervenwachstumsfaktor (NGF) oder ein Mutein davon mit der Aminosäuresequenz von SEQ ID Nr. 3, SEQ ID Nr. 4, SEQ ID Nr. 5 oder SEQ ID Nr. 6 zur Verwendung bei der Vorbeugung oder Behandlung von sensorineuralem Hörverlust bei einem Patienten, wobei der NGF oder das Mutein dem Patienten intranasal verabreicht wird.

2. NGF oder Mutein zur Verwendung nach Anspruch 1, wobei der sensorineurale Hörverlust nicht-genetischer Ätiologie ist.

3. NGF oder Mutein zur Verwendung nach Anspruch 2, wobei der sensorineurale Hörverlust nicht-genetischer Ätiologie durch Lärmbelastung, eine bakterielle oder virale Infektion, die Behandlung mit einem ototoxischen Medikament, eine Autoimmunerkrankung oder Alterung verursacht wird.

4. NGF oder Mutein zur Verwendung nach den Ansprüchen 1 bis 3, wobei es sich bei dem NGF um menschlichen NGF, bevorzugter um rekombinanten menschlichen NGF handelt.

5. NGF oder Mutein zur Verwendung nach Anspruch 4, wobei der menschliche NGF die Aminosäuresequenz von SEQ ID Nr. 1 oder SEQ ID Nr. 2 aufweist.

6. NGF oder Mutein zur Verwendung nach den Ansprüchen 1 bis 5, wobei der NGF oder das Mutein ein- bis dreimal täglich für einen Behandlungszeitraum zwischen 7 und 300 Tagen, vorzugsweise zwischen 60 und 240 Tagen, bevorzugter zwischen 100 und 200 Tagen verabreicht wird.

7. NGF oder Mutein zur Verwendung nach den Ansprüchen 1 bis 5, wobei die Menge an NGF pro Verabreichung zwischen 5 µg und 1 mg, bevorzugter zwischen 10 µg und 400 µg, noch bevorzugter zwischen 15 µg und 200 µg liegt.

8. Pharmazeutische Zusammensetzung, umfassend NGF oder Mutein davon mit der Aminosäuresequenz von SEQ ID Nr. 3, SEQ ID Nr. 4, SEQ ID Nr. 5 oder SEQ ID Nr. 6 und mindestens einen pharmazeutisch verträglichen Hilfsstoff zur Verwendung bei der Vorbeugung oder Behandlung von sensorineuralem Hörverlust bei einem Patienten, wobei die Zusammensetzung dem Patienten intranasal verabreicht wird.

9. Pharmazeutische Zusammensetzung zur Verwendung nach Anspruch 8, wobei der sensorineurale Hörverlust nicht-genetischer Ätiologie durch Lärmbelastung, eine bakterielle oder virale Infektion, die Behandlung mit einem ototoxischen Medikament, eine Autoimmunerkrankung oder Alterung verursacht wird.

10. Pharmazeutische Zusammensetzung zur Verwendung nach Anspruch 8 oder 9, wobei der NGF oder das Mutein davon in der Zusammensetzung in einer Konzentration zwischen 5 µg/ml und 1 mg/ml, bevorzugter zwischen 10 µg/ml und 400 µg/ml, noch bevorzugter zwischen 15 µg/ml und 200 µg/ml vorliegt.

11. Pharmazeutische Zusammensetzung zur Verwendung nach den Ansprüchen 8 bis 10, umfassend, vorzugsweise bestehend aus NGF oder Mutein davon, Natriumchlorid, Phosphatpuffer und Wasser.

12. Pharmazeutische Zusammensetzung zur Verwendung nach Anspruch 8 bis 11, umfassend, vorzugsweise bestehend aus:
- NGF oder Mutein davon mit der Aminosäuresequenz von SEQ ID Nr. 3, SEQ ID Nr. 4, SEQ ID Nr. 5 oder SEQ ID Nr. 6, vorzugsweise in einer Konzentration zwischen 5 µg/ml und 1 mg/ml, bevorzugter zwischen 10 µg/ml und 400 µg/ml, noch bevorzugter zwischen 15 µg/ml und 200 µg/ml,
- NaH2PO4 * H2O, vorzugsweise in einer Konzentration zwischen 5 und 8 mg/ml, bevorzugter von 6,9 mg/mL,
- NaCl, vorzugsweise in einer Konzentration zwischen 5 und 6,5 mg/ml, bevorzugter von 5,84 mg/ml,
- Poloxamer 188, vorzugsweise in einer Konzentration zwischen 0,05 % Gew./V. und 0,2 % Gew./V., bevorzugter von 0,1 % Gew./V.,
- L-Methionin, vorzugsweise in einer Konzentration zwischen 0,005 mg/ml und 0,02 mg/ml, bevorzugter von 0,01 mg/ml,
- optional n-Dodecyl-13-D-Maltosid, vorzugsweise in einer Konzentration zwischen 0,1 % Gew./V. und 1 % Gew./V., bevorzugter von 0,5 % Gew./V., und/oder Glycerin, vorzugsweise in einer Konzentration zwischen 0,05 % Gew./V. und 0,2 % Gew./V., bevorzugter von 0,1 % Gew./V.,
- Wasser.

13. Pharmazeutische Zusammensetzung zur Verwendung nach den Ansprüchen 8 bis 12, wobei es sich bei dem NGF um menschlichen NGF, bevorzugter um rekombinanten menschlichen NGF handelt.

14. Pharmazeutische Zusammensetzung zur Verwendung nach Anspruch 13, wobei der menschliche NGF die Aminosäuresequenz von SEQ ID Nr. 1 oder SEQ ID Nr. 2 aufweist.

## Revendications

1. Facteur de croissance nerveuse (NGF) ou une de ses mutéines ayant la séquence d'acides aminés de SEQ ID No. 3, SEQ ID No. 4, SEQ ID No. 5 ou SEQ ID No. 6, à utiliser dans la prévention ou le traitement de la perte auditive neurosensorielle chez un sujet, dans lequel ledit NGF ou ladite mutéine est administré(e) par voie intranasale audit sujet.

2. NGF ou mutéine à utiliser selon la revendication 1, dans lequel ladite perte auditive neurosensorielle est d'étiologie non génétique.

3. NGF ou mutéine à utiliser selon la revendication 2, dans lequel ladite perte auditive neurosensorielle d'étiologie non génétique est causée par une exposition au bruit, une infection bactérienne ou virale, le traitement par un médicament ototoxique, une maladie auto-immune ou le vieillissement.

4. NGF ou mutéine à utiliser selon les revendications 1 à 3, dans lequel ledit NGF est un NGF humain, plus préférablement un NGF humain recombinant.

5. NGF ou mutéine à utiliser selon la revendication 4, dans lequel ledit NGF humain a la séquence d'acides aminés de SEQ ID No. 1 ou SEQ ID No. 2.

6. NGF ou mutéine à utiliser selon les revendications 1 à 5, dans lequel ledit NGF ou ladite mutéine est administré(e) de une à trois fois par jour pendant une période de traitement comprise entre 7 et 300 jours, de préférence entre 60 et 240 jours, plus préférablement entre 100 et 200 jours.

7. NGF ou mutéine à utiliser selon les revendications 1 à 5, dans lequel la quantité de NGF par administration est comprise entre 5 µg et 1 mg, plus préférablement entre 10 µg et 400 µg, encore plus préférablement entre 15 µg et 200 µg.

8. Composition pharmaceutique comprenant du NGF ou sa mutéine ayant la séquence d'acides aminés de SEQ ID No. 3, SEQ ID No. 4, SEQ ID No. 5 ou SEQ ID No. 6, et au moins un excipient pharmaceutiquement acceptable, à utiliser dans la prévention ou le traitement de la perte auditive neurosensorielle chez un sujet, dans laquelle ladite composition est administrée par voie intranasale audit sujet.

9. Composition pharmaceutique à utiliser selon la revendication 8, dans laquelle ladite perte auditive neurosensorielle d'étiologie non génétique est causée par une exposition au bruit, une infection bactérienne ou virale, le traitement par un médicament ototoxique, une maladie auto-immune ou le vieillissement.

10. Composition pharmaceutique à utiliser selon la revendication 8 ou 9, dans laquelle ledit NGF ou sa mutéine est présent(e) dans la composition à une concentration comprise entre 5 µg/ml et 1 mg/ml, plus préférablement entre 10 µg/ml et 400 µg/ml, encore plus préférablement entre 15 µg/ml et 200 µg/ml.

11. Composition pharmaceutique à utiliser selon les revendications 8 à 10, comprenant, de préférence, constitué du NGF ou de sa mutéine, du chlorure de sodium, d'un tampon phosphate et de l'eau.

12. Composition pharmaceutique à utiliser selon les revendications 8 à 11, comprenant, de préférence constituée de :
- NGF ou sa mutéine ayant la séquence d'acides aminés de SEQ ID No. 3, SEQ ID No. 4, SEQ ID No. 5 ou SEQ ID No. 6, de préférence à une concentration comprise entre 5 µg/ml et 1 mg/ml, plus préférablement entre 10 µg/ml et 400 µg/ml, encore plus préférablement entre 15 µg/ml et 200 µg/ml,
- NaH2PO4 * H2O, de préférence à une concentration comprise entre 5 et 8 mg/ml, plus préférablement de 6,9 mg/ml,
- NaCl, de préférence à une concentration comprise entre 5 et 6,5 mg/ml, plus préférablement de 5,84 mg/ml,
- Poloxamère 188, de préférence à une concentration comprise entre 0,05 % p/v et 0,2 % p/v, plus préférablement à 0,1 % p/v,
- L-méthionine, de préférence à une concentration comprise entre 0,005 mg/ml et 0,02 mg/ml, plus préférablement à 0,01 mg/ml,
- optionnellement, le n-Dodécyl-13-D-maltoside, de préférence à une concentration comprise entre 0,1 % p/v et 1 % p/v, plus préférablement de 0,5 % p/v, et/ou le glycérol, de préférence à une concentration comprise entre 0,05 % p/v et 0,2 % p/v, plus préférablement de 0,1 % p/v,
- l'eau.

13. Composition pharmaceutique à utiliser selon les revendications 8 à 12, dans laquelle ledit NGF est un NGF humain, plus préférablement un NGF humain recombinant.

14. Composition pharmaceutique à utiliser selon la revendication 13, dans laquelle ledit NGF humain présente la séquence d'acides aminés de SEQ ID No. 1 ou SEQ ID No. 2.
